# EUROPEAN PATENT APPLICATION

(11) **EP 3 156 023 A1**
(43) Date of publication of application: **19.04.2017**
(21) Application number: 15189757.6
(22) Date of filing: 14.10.2015
(51) Int. Cl.: A61F 13/62, A44B 18/00

(54) **NONWOVEN FABRIC FASTENING STRUCTURE**

(71) Applicant: Cheng, Yuan-Long, Tainan City (TW)
(72) Inventor: Cheng, Yuan-Long, Tainan City (TW)
(74) Representative: Pallini Gervasi, Diego

(57) **Abstract**

The present invention relates to a nonwoven fabric fastening structure (1). The nonwoven fabric fastening structure (1) comprises a fastening body (10) and a nonwoven fabric (20). The fastening body (10) comprises a fastening end (11) and multiple fastening units (12). The fastening units (12) are formed on a surface of the fastening end (11). The nonwoven fabric (20) comprises a surface and a pattern. The pattern comprises multiple grooves (22). The grooves (22) are formed on the surface of the nonwoven fabric (20). The fastening body (10) is selectively combined with the nonwoven fabric (20). When the fastening body (10) is combined with the nonwoven fabric (20), the grooves (22) formed on the surface of the nonwoven fabric (20) increase the bonding strength between the fastening body (10) and the nonwoven fabric (20) and prevent the fastening body (10) from moving.

## Description

### 1. Field of the Invention

The present invention relates to a nonwoven fabric fastening structure, especially to a nonwoven fabric fastening structure applying to diapers.

### 2. Description of the Prior Art(s)

Diaper is an essential item for a baby. To prevent an uncomfortable sticky feeling while the baby is wearing the diaper, the ventilation of the diaper is an important factor to be considered. Hence, nonwoven fabric having great ventilation becomes the main component of diapers.

Many methods for manufacturing nonwoven fabric for diapers are developed, for example, Taiwan patent M445589 discloses a method for manufacturing nonwoven fabric. In said method, multiple sheets of cottons are stacked in a stepped pattern by controlling the transportation speed of the cottons; the cottons are heated and then shrank to form a nonwoven fabric.

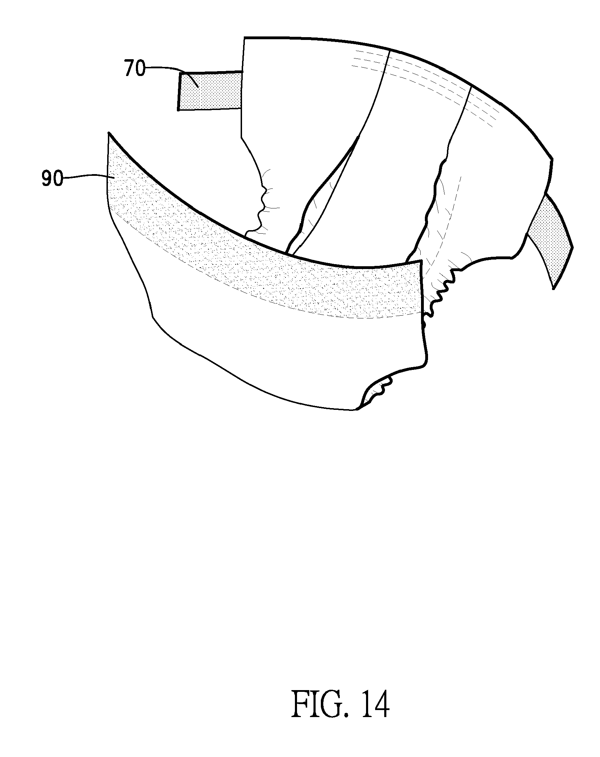

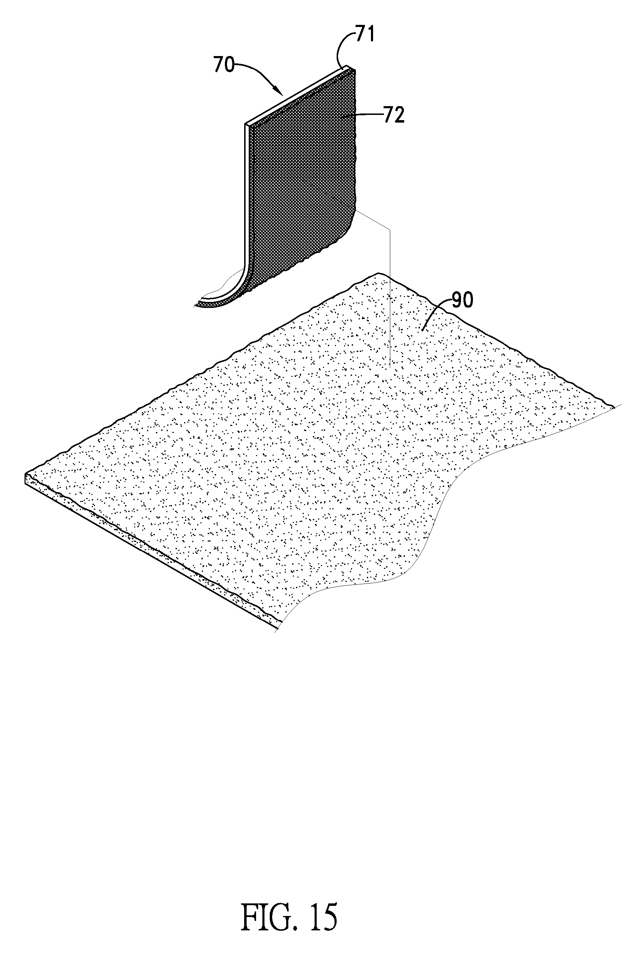

With reference to Fig. 14 and Fig. 15, a conventional diaper comprises a fastening portion 70. The fastening portion 70 comprises a body 71 and a sticky piece 72. The sticky piece 72 is attached on the body 71 of the fastening portion 70. The fastening portion 70 can selectively stick to the surface 90 of nonwoven fabric of the conventional diaper to fix the conventional diaper by the sticky piece 72. However, the sticky piece 72 of the fastening portion 70 reduces the local air permeability of the conventional diaper, and thus causes the discomfort in wearing.

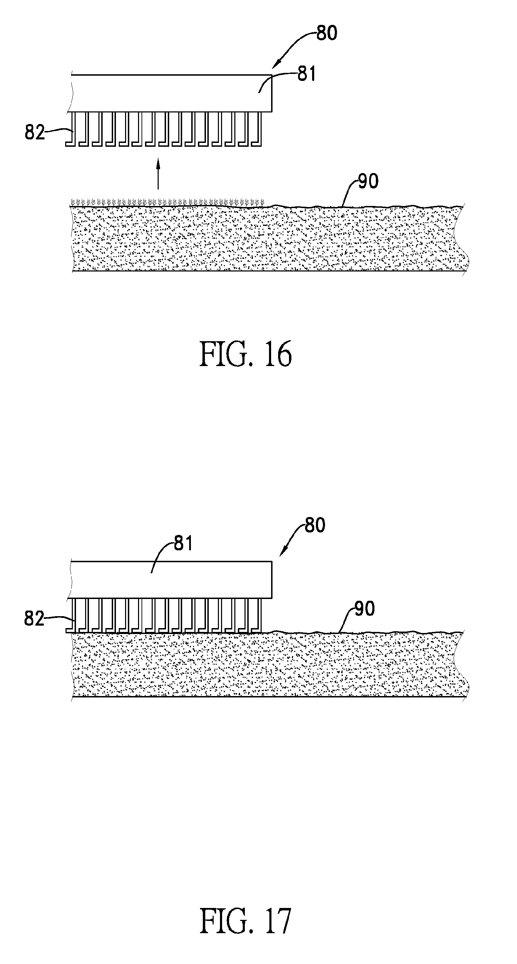

To overcome the problem caused from the sticky piece 72, a new kind of fastening portion is developed. With referenced to Fig. 16 and Fig. 17, the fastening portion 80 comprises a body 81. Multiple hooks 82 are formed on the body 81 of the fastening portion 80. The fastening portion 80 can selectively attach to the surface 90 of the conventional diaper to fix the conventional diaper by the hooks 82 without reducing the local air permeability of the conventional diaper. However, the surface 90 of the nonwoven fabric of the conventional diaper is flat; therefore the hooks 82 cannot tightly attach the surface of the nonwoven fabric, that is to say, the hooks 82 can be easily moved on the surface of the nonwoven fabric. Moreover, repeated use of the fastening portion 80 causes the surface 90 of the conventional diaper to become hairy, thereby weakening the bonding between the fastening portion 80 and the surface 90 of the conventional diaper.

To overcome the shortcomings, the present invention provides a nonwoven fabric fastening structure to mitigate or obviate the aforementioned problems.

The objective of the present invention is providing a nonwoven fabric fastening structure to improve the bonding strength between the hooks and the surface of the nonwoven fabric on the premise of maintaining the ventilation of the nonwoven fabric.

To achieve the abovementioned objective, the present invention provides a nonwoven fabric fastening structure. The nonwoven fabric fastening structure comprises a fastening body and a nonwoven fabric. The fastening body comprises a fastening end and multiple fastening units. The fastening units are formed on a surface of the fastening end. The nonwoven fabric comprises a surface and a pattern. The pattern comprises multiple grooves. The grooves are formed on the surface of the nonwoven fabric. The fastening body is selectively combined with the nonwoven fabric.

When the fastening body is combined with the nonwoven fabric, the grooves formed on the surface of the nonwoven fabric increase the bonding strength between the fastening body and the nonwoven fabric, and prevent the fastening body from moving on the nonwoven fabric.

Preferably, each groove is strip-shaped and the grooves are parallel to each other. More preferably, a distance between any two neighboring grooves ranges from 0.7 mm to 3.0 mm, inclusive.

Preferably, a width of each one of the grooves ranges from 0.5 mm to 0.8 mm.

Preferably, multiple bosses are formed between the grooves, each groove has a bottom surface, each boss has a top surface, and a vertical distance between the top surface and the bottom surface ranges from 0.2 mm to 0.8 mm, inclusive.

Preferably, a thickness of the nonwoven fabric ranges from 0.3 mm to 1.3 mm.

More preferably, a proportion of the vertical distance between the top surface and the bottom surface and the thickness of the nonwoven fabric ranges from 0.15 to 0.88.

More preferably, a proportion of the vertical distance between the top surface and the bottom surface and the distance between any two neighboring bosses ranges from 0.26 to 1.06.

Preferably, a proportion of an area of the bottom surfaces of the grooves and an area of the surface of the nonwoven fabric ranges from 31 % to 52%. When the proportion of the area of the bottom surfaces of the grooves and the area of the surface of the nonwoven fabric is less than 31 % or greater than 52%, the bonding strength between the fastening body and the nonwoven fabric is decreased.

Alternatively, the pattern comprises multiple lines, the lines are parallel to each other, each line consists of the grooves, a spaced interval is formed between any two neighboring grooves, and each groove is rectangular.

Alternatively, each groove is dot-shaped and the grooves are arranged separately on the surface of the nonwoven fabric.

Preferably, each fastening unit of the fastening units comprises a linking part and a hooking part, the linking part has two ends, one of the ends of the linking part is connected to the surface of the fastening end, the other end of the linking part is connected to the hooking part. The fastening body is combined with the nonwoven fabric by the hooking parts.

Preferably, the hooking part is strip-shaped, the linking part is strip-shaped, the other end of the linking part is connected to a middle of the hooking part, and the hooking part is divided into two equal regions by the linking part.

Alternatively, the hooking part is cross -shaped and has two faces, the linking part is column-shaped, and the other end of the linking part is connected to a center of one of the faces of the hooking part.

Alternatively, the hooking part is strip-shaped and has two ends, the linking part is strip-shaped, and the other end of the linking part is connected to one of the ends of the hooking part.

Other objectives, advantages and novel features of the invention will become more apparent from the following detailed description when taken in conjunction with the accompanying drawings.

### In the drawings:

Fig. 1 is a schematic view of a nonwoven fabric fastening structure of Embodiment 1 of the present invention;
Fig. 2 is a side view of a fastening body of the nonwoven fabric fastening structure of Embodiment 1 of the present invention;
Fig. 3A is a bottom view of the fastening body of the nonwoven fabric fastening structure of Embodiment 1 of the present invention;
Fig. 3B is an enlarged bottom view of fastening units in Fig. 3A;
Fig. 4 is a side view of the fastening body and the nonwoven fabric of the nonwoven fabric fastening structure of Embodiment 1 of the present invention;
Fig. 5 is a side view of the fastening body firmly fixed with the nonwoven fabric;
Fig. 6 is a schematic view of a nonwoven fabric fastening structure of Embodiment 2 of the present invention;
Fig. 7 is a side view of a fastening body of the nonwoven fabric fastening structure of Embodiment 2 of the present invention;
Fig. 8A is a bottom view of the fastening body of the nonwoven fabric fastening structure of Embodiment 2 of the present invention;
Fig. 8B is an enlarged bottom view of the fastening body of the nonwoven fabric fastening structure of Embodiment 2 of the present invention;
Fig. 9 is a schematic view of a nonwoven fabric fastening structure of Embodiment 3 of the present invention;
Fig. 10 is a side view of a fastening body of the nonwoven fabric fastening structure of Embodiment 3 of the present invention;
Fig. 11A is a bottom view of the fastening body of the nonwoven fabric fastening structure of Embodiment 3 of the present invention;
Fig. 11B is an enlarged bottom view of the fastening body of the nonwoven fabric fastening structure of Embodiment 3 of the present invention;
Fig. 12A is a schematic view of Experimental Embodiment 1 when the nonwoven fabric fastening structure of Embodiment 2 of the present invention was pinned by a thumbtack and was hanged a counterpoise;
Fig. 12B is a schematic view of Experimental Embodiment 1 when measuring the peeling strength of the nonwoven fabric fastening structure of Embodiment 2 of the present invention with a tension testing machine;
Fig. 13 is a schematic view of Experimental Embodiment 2 when measuring the shear strength of the nonwoven fabric fastening structure of Embodiment 2 of the present invention with a tension testing machine;
Fig. 14 is a schematic view of a conventional diaper;
Fig. 15 is a schematic view of a fastening portion and a surface of the conventional diaper;
Fig. 16 is a side view of a fastening portion and a surface of a conventional diaper; and
Fig. 17 is a side view of the fastening portion fixed with the surface of the conventional diaper.

### Embodiment 1

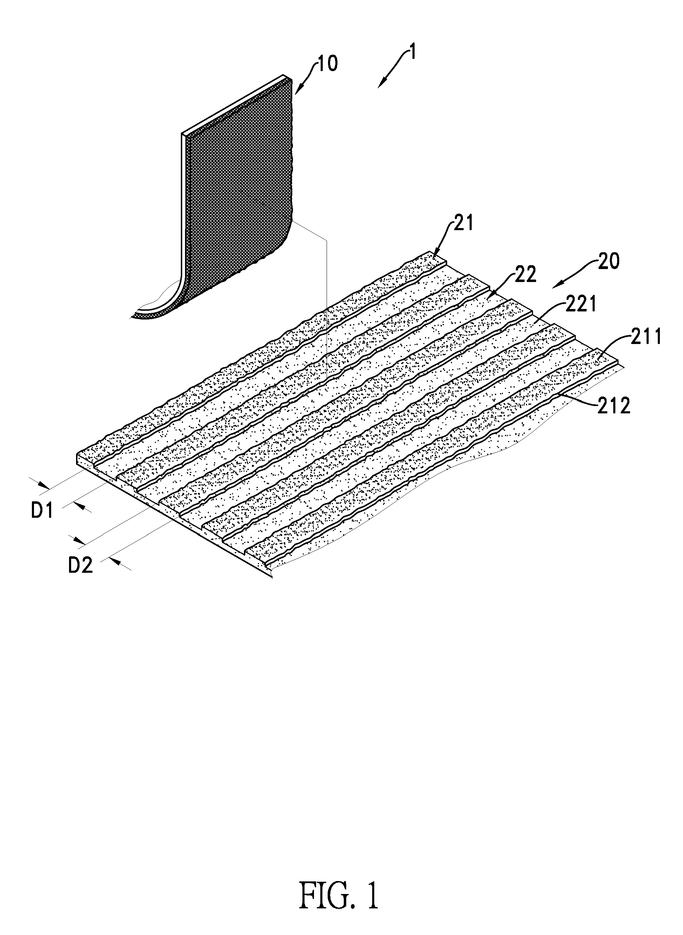

With reference to Fig. 1, this embodiment provides a nonwoven fabric fastening structure 1. The nonwoven fabric fastening structure 1 comprises a fastening body 10 and a nonwoven fabric 20.

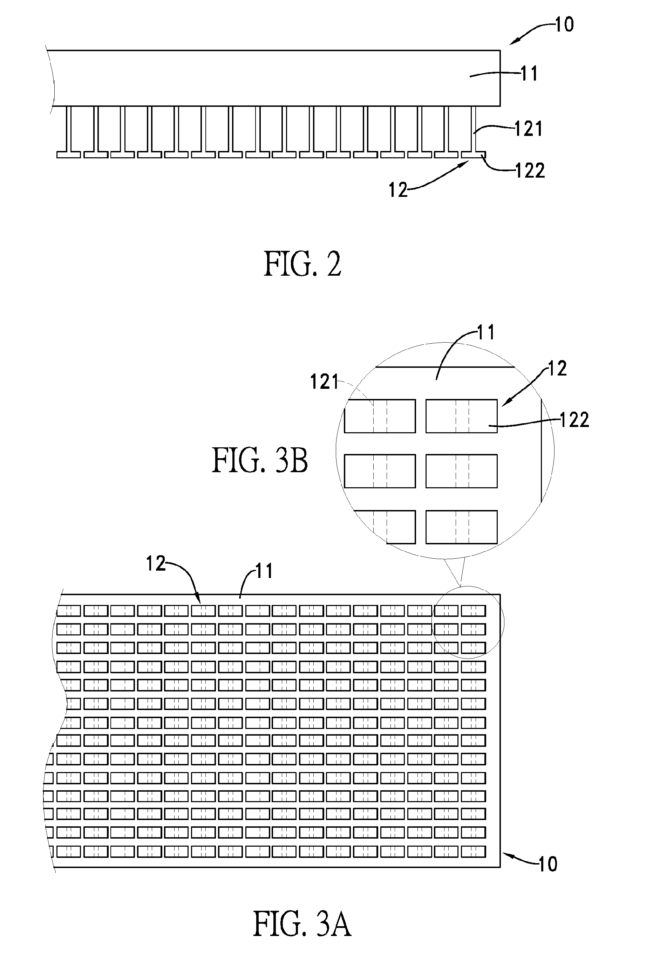

With reference to Fig. 1 to Fig. 3B, the fastening body 10 is selectively fixed with the nonwoven fabric 20 and comprises a fastening end 11 and multiple fastening units 12. The fastening units 12 are formed on a surface of the fastening end 11. Each fastening unit 12 comprises a linking part 121 and a hooking part 122 connected to the linking part 121. Specifically, in the present embodiment, both the linking part 121 and the hooking part 122 are strip-shaped. The linking part 121 has two ends, one of the ends of the linking part 121 is connected to the surface of the fastening end 11, and the other end of the linking part 121 is connected to a middle of the hooking part 122. The strip-shaped hooking part 122 is divided into two equal regions by the linking part 121. The two equal regions of the hooking part 122 are respectively located at two faces of the other end of the linking part 121.

The nonwoven fabric 20 is prepared by the following method. A raw cloth is provided and fed to a cloth extension device. The detailed configuration of the cloth extension device can be found in Taiwan patent I399469. The raw cloth is transported to a set of rolling wheels of the cloth extension device. The set of rolling wheels comprises a first rolling wheel and a second rolling wheel; the first rolling wheel and the second rolling wheel respectively rotate clockwise and counterclockwise. The raw cloth is transported to a site between the first rolling wheel and the second rolling wheel and is rolled and pressed by the first rolling wheel and the second rolling wheel to form a pressed cloth.

The pressed cloth is then transported to a furnace of the cloth extension device. The temperature of the furnace is set between the softening point and the melting point of the raw cloth. The pressed cloth is then heated in the furnace to form a heated cloth. By means of heating the pressed cloth, the modulus of the fibers of the heated cloth is lower than that of the pressed cloth; that is to say, the heated cloth is softer than the pressed cloth. Besides, the heated cloth is sticky and shapeable.

The heated cloth is then transported to a set of stationary wheels of the cloth extension device. The set of stationary wheels comprises two stationary wheels. The stationary wheels respectively rotate clockwise and counterclockwise. The rotation speed of the stationary wheels is faster than the first rolling wheel and the second rolling wheel. The heated cloth is transported to a site between the stationary wheels and is rolled and pressed by the stationary wheels to form an un-patterned cloth. Due to the unequal rotation speeds between the set of stationary wheels and the set of rolling wheels, the un-patterned cloth is extended while rolling by the stationary wheels.

The un-patterned cloth is glued with a greige and then is transported to a hot pressing wheel having a patterned surface and is pressed by the hot pressing wheel at 130 °C to 150°C, to form the nonwoven fabric 20 with a pattern. Said nonwoven fabric 20 is an elastic nonwoven fabric.

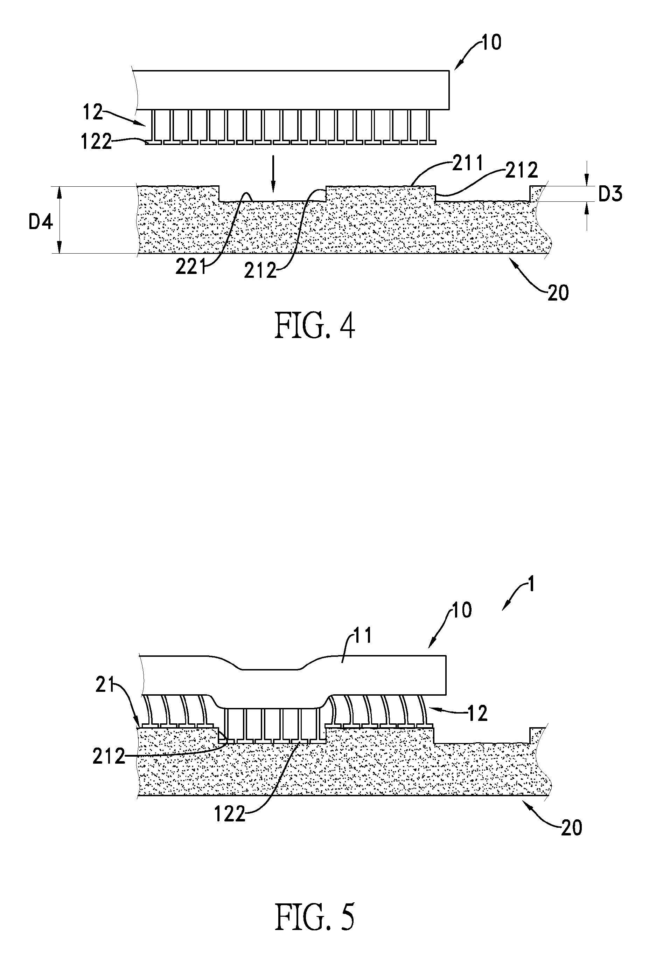

With reference to Figs. 1 and 4, the nonwoven fabric 20 is consisted of fibers. The nonwoven fabric 20 comprises a surface and the pattern. The pattern is formed on the surface of the nonwoven fabric 20. The pattern comprises multiple bosses 21 and multiple grooves 22. The bosses 21 are formed on the surface of the nonwoven fabric 20. Each boss 21 is strip-shaped. The bosses 21 are parallel to each other and the grooves 22 are formed among the bosses 21. Specifically, each groove 22 is strip-shaped. The grooves 22 are parallel to each other. The bosses 21 and the grooves 22 are parallel to the fibers consisting of the nonwoven fabric 20. A distance D1 between any two neighboring bosses is 0.75 mm; that is to say, a width of one groove of the grooves is 0.75 mm. A distance D2 between any two neighboring grooves is 0.7 mm; that is to say, a width of one boss of the bosses is 0.7 mm. Each boss 21 has a top surface 211 and two side walls 212 and two sides of the top surface 211 are connected to the side walls 212 respectively. Each groove 22 has a bottom surface 221. A vertical distance D3 between the top surface 211 and the bottom surface 221 is 0.5 mm. A thickness D4 of the nonwoven fabric 20 is 0.8 mm. The thickness D4 of the nonwoven fabric 20 is indicated as a vertical distance between the top surface of the bosses 21 and a bottom surface of the nonwoven fabric 20. The bottom surface of the nonwoven fabric 20 is opposite the surface of the nonwoven fabric 20. A proportion of the vertical distance D3 between the top surface 211 of the bosses 21 and the bottom surface 221 of the grooves 22 and the thickness D4 of the nonwoven fabric 20 is 0.625. A proportion of the vertical distance D3 between the top surface 211 and the bottom surface 221 and the distance D1 between any two neighboring bosses is 0.66.

The nonwoven fabric fastening structure 1 can be applied on a diaper. The nonwoven fabric 20 can be used as the surface of the diaper; that is to say, the surface of the diaper is to have the bosses 21 and the grooves 22. The fastening body 10 can be used as the fastening portion of the diaper.

With reference to Figs. 4 and 5, when the fastening body 10 is fixed with the nonwoven fabric 20, which means the nonwoven fabric fastening structure 1 is in a fixed state, the fastening end 11 is attached on the surface of the nonwoven fabric 20 and the fastening units 12 are hooked on the side walls 212 of the bosses 21 by the hooking parts 122. Because the fastening units 12 are hooked on the side walls 212 of the bosses 21, the fastening body 10 can be firmly fixed with the nonwoven fabric 20 without moving.

### Embodiment 2

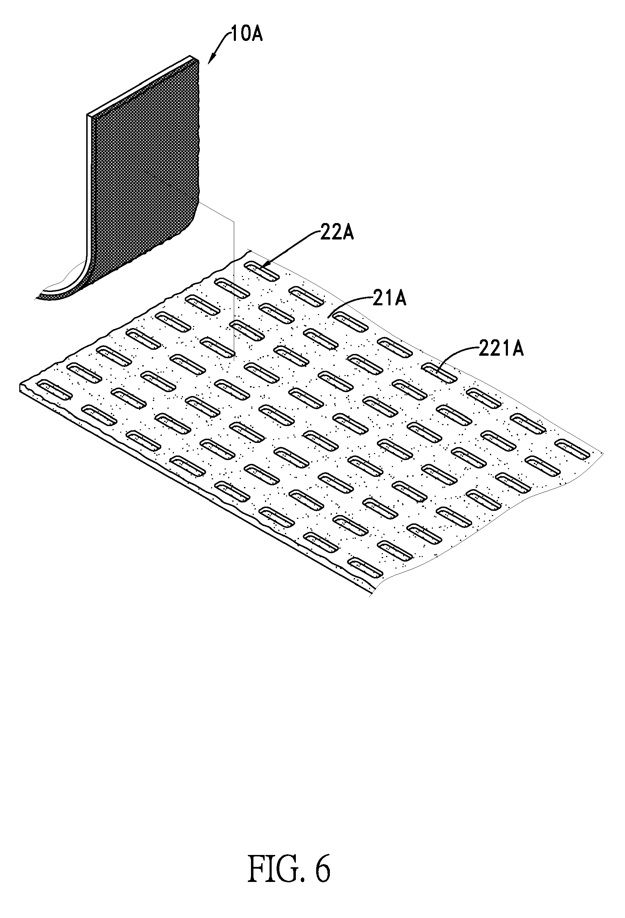

With reference to Figs. 6 to 8B, this embodiment provides a nonwoven fabric fastening structure 1A and the configuration of the nonwoven fabric fastening structure 1A of this embodiment is similar to the configuration of the nonwoven fabric fastening structure 1 of Embodiment 1. The differences between the nonwoven fabric fastening structure 1A of this embodiment and the nonwoven fabric fastening structure 1 of Embodiment 1 are as follows.

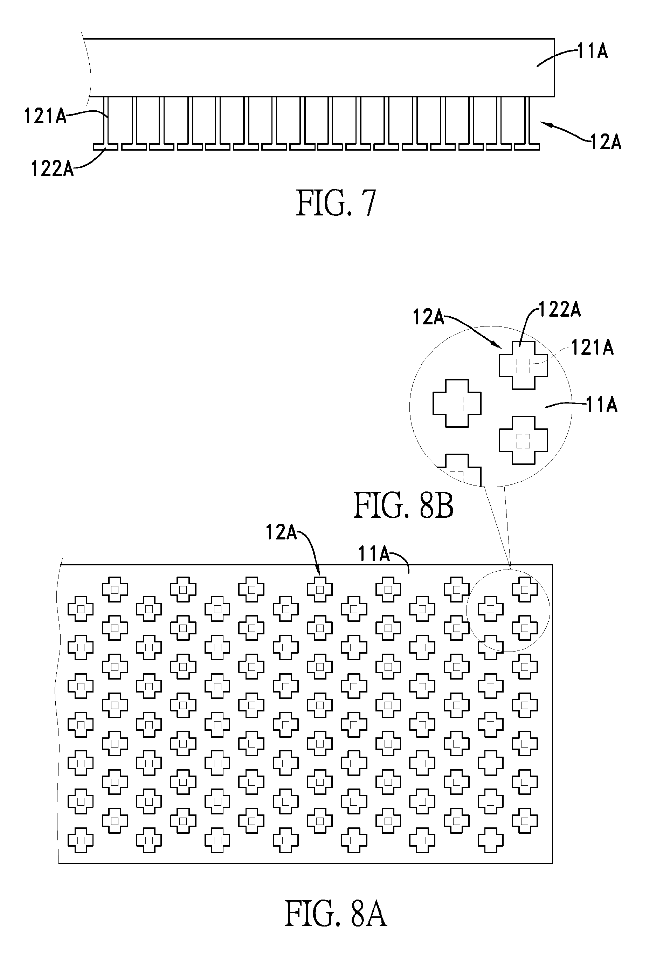

With reference to Figs. 7, 8A, and 8B, each fastening unit 12A comprises a linking part 121A and a hooking part 122A connected to the linking part 121A. The hooking part 122A is cross-shaped and has two faces. The linking part 121A is column-shaped and the linking part 121A has two ends. One of the ends of the linking part 121A is connected to the surface of the fastening end 11A. The other end of the linking part 121A is connected to a center of one of the faces of the hooking part 122A.

With reference to Fig. 6, the pattern comprises multiple lines. The lines are formed on the surface of the nonwoven fabric 20A and are parallel to each other. Each line consists of multiple grooves 22A and a spaced interval is formed between any two neighboring grooves 22A. Each groove 22A is rectangular. A boss 21A is formed among the grooves 22A; in other words, the boss 21A is formed among the lines and the intervals between any two neighboring grooves 22A. Specifically, when an area of surface of the nonwoven fabric 20A is 50 mm x 50 mm, a proportion of an area of the bottom surfaces 221A of the grooves 22A and the area of surface of the nonwoven fabric 20A is 31.5%.

When the fastening body 10A is fixed with the nonwoven fabric 20A, the fastening units 12A can be placed into the grooves 22A among the boss 21A and the hooking part 122A are hooked on side walls of the boss 21A. The nonwoven fabric fastening structure 1A in this embodiment improves the bonding strength between the fastening body 10A and the nonwoven fabric 20A.

### Embodiment 3

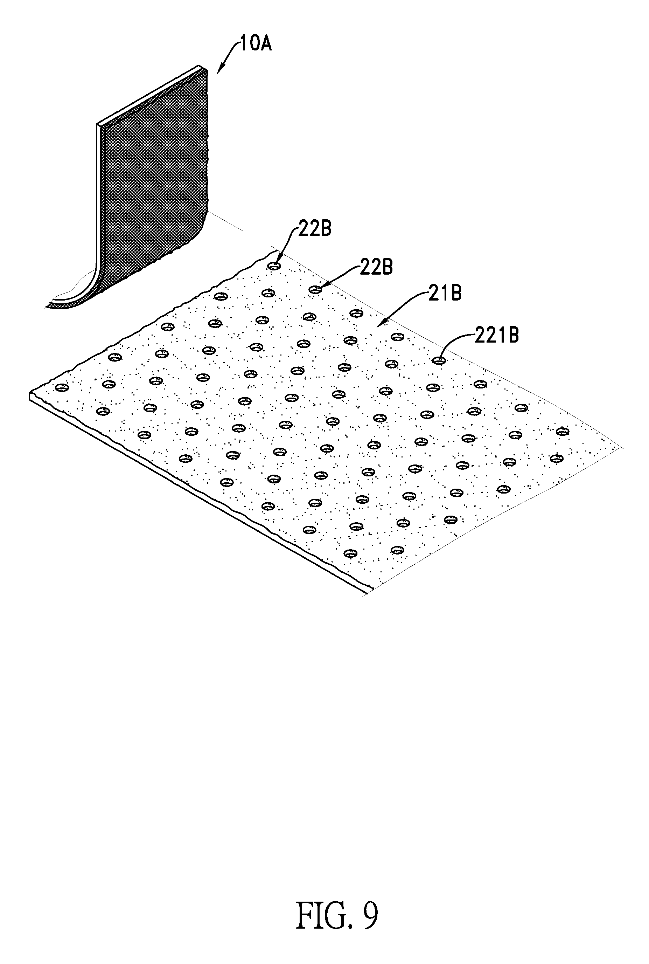

With reference to Figs. 9 to 11B, this embodiment provides a nonwoven fabric fastening structure 1 B and the configuration of the nonwoven fabric fastening structure 1B of this embodiment is similar to the configuration of the nonwoven fabric fastening structure 1 of Embodiment 1. The differences between the nonwoven fabric fastening structure 1B of this embodiment and the nonwoven fabric fastening structure 1 of Embodiment 1 are as follows.

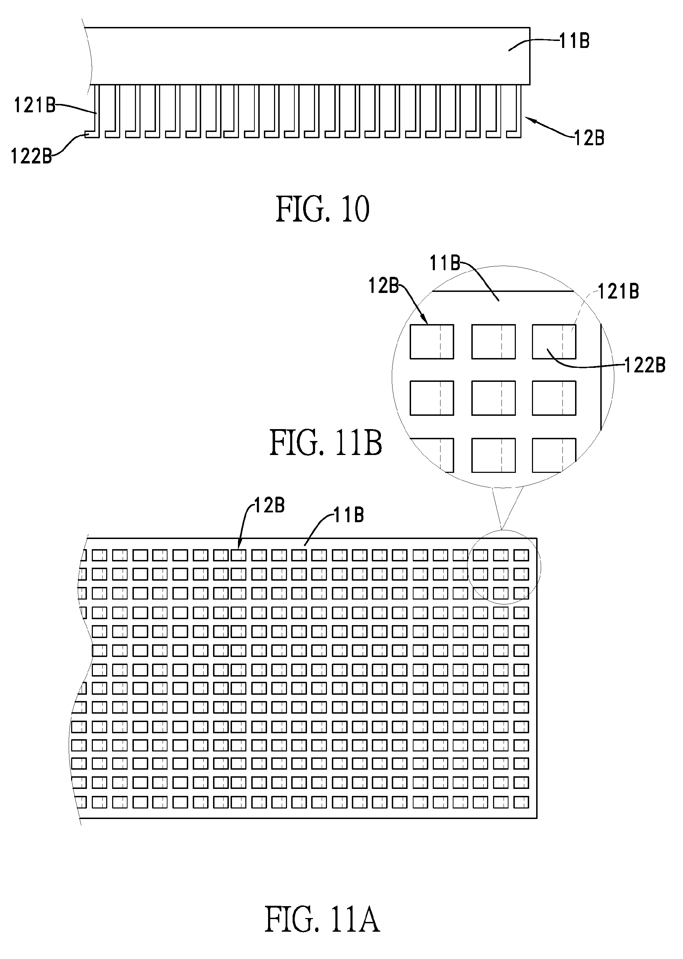

With reference to Figs. 10, 11A, and 11B, each fastening unit 12B comprises a linking part 121B and a hooking part 122B connected to the linking part 121B. The hooking part 12B is strip-shaped and has two ends. The linking part 121B is strip-shaped and the linking part 121B has two ends. One of the ends of the linking part 121B is connected to the surface of the fastening end 11B. The other end of the linking part 121B is connected to one of the ends of the hooking part 122B.

With reference to Fig. 9, the pattern comprises the grooves 22B. Each groove 22B is dot-shaped. The grooves 22B are arranged separately on the surface of the nonwoven fabric 20B. A boss 21B is formed among the grooves 22B. Specifically, when an area of surface of the nonwoven fabric 20A is 50 mm x 50 mm, a proportion of an area of the bottom surfaces 221B of the grooves 22B and the area of surface of the nonwoven fabric 20B is 51.5%.

When the fastening body 10B is fixed with the nonwoven fabric 20B, the fastening units 12B can be placed into the grooves 22B among the boss 21B and the hooking part 122B are hooked on side walls of the boss 21B. The nonwoven fabric fastening structure 1B in this embodiment improves the bonding strength between the fastening body 10B and the nonwoven fabric 20B.

### Experimental Embodiment 1

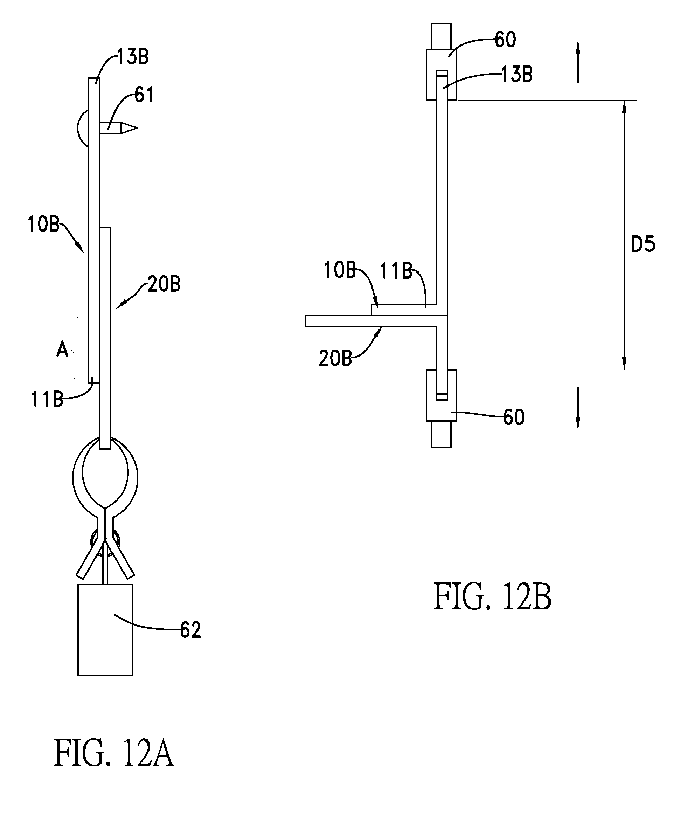

With reference to Fig. 12A and Fig. 12B, peeling strengths of the nonwoven fabric fastening structure 1B (Configuration 1) and the fastening portion 80 with a nonwoven fabric described in the prior art (Configuration 2) were measured respectively by a tension testing machine having two parts 60. The measuring method was as follows.

The fastening body 10B has the fastening end 11B and a clamping end 13B opposite the fastening end 11B. The fastening end 11B was attached on the surface of the nonwoven fabric 20B (65 g) to form a contacting area A. The contacting area A is 30 mm x 30 mm. The contacting area A was pressed by a roller (2 kg) twice. The clamping end 13B of the fastening body 10B was pinned on a wall by a thumbtack 61. One end of the nonwoven fabric 20B was hanged a counterpoise 62 (1 kg) for 5 seconds and then removed the thumbtack 61 and the counterpoise 62.

Two parts 60 of the tension testing machine were placed separately at a distance D5, 40 mm from each other. The fastening body 10B was bent into a right angle. The clamping end 13B of the fastening body 10B was clamped by one of the two parts 60 of the tension testing machine. The nonwoven fabric 20B was bent into a right angle and the other end of the nonwoven fabric 20B was clamped by the other part 60 of the tension testing machine.

Then, the two parts 60 of the tension testing machine were moved conversely at a speed 300 mm/min to peel the fastening body 10B and the nonwoven fabric 20B. The peeling strength of the fabric fastening structure 1B was measured three times and was recorded in Table 1.

The peeling strength of the fastening portion 80 with the nonwoven fabric described in prior art was measured in the same method three times and was recorded in Table 1.

### Experimental Embodiment 2

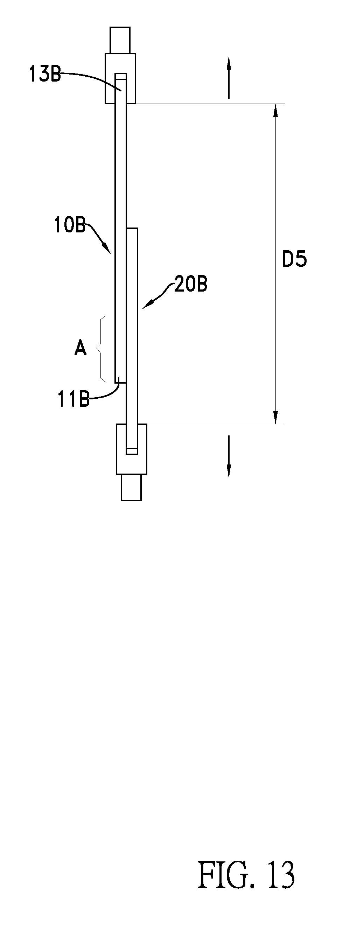

With reference to Fig. 13, shear strengths of the nonwoven fabric fastening structure 1B (Configuration 1) and the fastening portion 80 with a nonwoven fabric described in prior art (Configuration 2) were measured respectively by a tension testing machine having two parts 60. The measuring method was as follows.

The fastening body 10B has the fastening end 11B and a clamping end 13B opposite the fastening end 11B. The fastening end 11B was attached on the surface of the nonwoven fabric 20B (65 g) to form a contacting area A. The contacting area A is 30 mm x 30 mm. The contacting area A was pressed by a roller (2 kg) twice. The clamping end 13B of the fastening body 10B was pinned on a wall by a thumbtack 61. One end of the nonwoven fabric 20B was hanged a counterpoise 62 (1 kg) for 5 seconds and then removed the thumbtack 61 and the counterpoise 62.

Two parts 60 of the tension testing machine were placed separately at a distance D6, 50 mm from each other. The clamping end 13B of the fastening body 10B was clamped by one of the two parts 60 of the tension testing machine. The end of the nonwoven fabric 20B was clamped by the other part 60 of the tension testing machine.

Then, the two parts 60 of the tension testing machine were moved conversely at a speed 300 mm/min to shear the fastening body 10B and the nonwoven fabric 20B. The shear strength of the fabric fastening structure 1B was measured three times and was recorded in Table 1.

The shear strength of the fastening portion 80 with the nonwoven fabric described in prior art was measured in the same method three times and was recorded in Table 1.

**Table 1: the peeling strength and shear strength of the nonwoven fabric fastening structure 1B and the fastening portion 80 with the nonwoven fabric described in prior art**

| | Peeling strength (N) | | Shear strength (N) | |
|---|---|---|---|---|
| | Configuration 1 | Configuration 2 | Configuration 1 | Configuration 2 |
| Maximum | 2.73 | 1.35 | 58.60 | 18.90 |
| Minimum | 2.13 | 0.80 | 45.25 | 14.13 |
| Average | 2.46 | 1.04 | 51.93 | 15.90 |

With reference to table 1, the peeling strength and shear strength of the nonwoven fabric fastening structure 1B fixed with the nonwoven fabric are respectively greater than those of the fastening portion 80 fixed with the nonwoven fabric as described in prior art; that is to say, the combination of the fastening body 10B and the nonwoven fabric 20B of the nonwoven fabric fastening structure 1B is stronger than the combination of the fastening portion 80 with the nonwoven fabric described in prior art. Therefore, the fastening body 10B can be firmly fixed with the nonwoven fabric 20B without moving. The nonwoven fabric fastening structure not only provides an improved bonding strength and also ensures a required local air permeability, thereby improving the applicability and comfort of the diaper.

## Claims

1. A nonwoven fabric fastening structure (1) **characterized in that** the nonwoven fabric fastening structure (1) comprising:
a fastening body (10) comprising a fastening end (11) and multiple fastening units (12), the fastening units (12) formed on a surface of the fastening end (11);
a nonwoven fabric (20) comprising a surface and a pattern, the pattern comprising multiple grooves (22), the grooves (22) formed on the surface of the nonwoven fabric (20);
wherein the fastening body (10) is selectively combined with the nonwoven fabric (20).

2. The nonwoven fabric fastening structure (1) as claimed in claim 1, wherein each groove (22) is strip-shaped and the grooves (22) are parallel to each other.

3. The nonwoven fabric fastening structure (1) as claimed in claim 1, wherein the pattern comprises multiple lines, the lines are parallel to each other, each line consists of the grooves (22), a spaced interval is formed between any two neighboring grooves (22), and each groove (22) is rectangular.

4. The nonwoven fabric fastening structure (1) as claimed in claim 1, wherein each groove (22) is dot-shaped and the grooves (22) are arranged separately on the surface of the nonwoven fabric (20).

5. The nonwoven fabric fastening structure (1) as claimed in claim 1, wherein each groove (22) has a bottom surface (221), and a proportion of an area of the bottom surfaces (221) of the grooves (22) and an area of the surface of the nonwoven fabric (20) ranges from 31 % to 52%.

6. The nonwoven fabric fastening structure (1) as claimed in claim 1, wherein multiple bosses (21) are formed between the grooves (22), each groove (22) has a bottom surface (221), each boss (21) has a top surface (211), and a proportion of a vertical distance (D3) between the top surface (211) and the bottom surface (221) and a thickness (D4) of the nonwoven fabric (20) ranging from 0.15 to 0.88.

7. The nonwoven fabric fastening structure (1) as claimed in claim 1, wherein each fastening unit (12) comprises a linking part (121) and a hooking part (122), the linking part (121) has two ends, one of the ends of the linking part (121) is connected to the surface of the fastening end (11), the other end of the linking part (121) is connected to the hooking part (122).

8. The nonwoven fabric fastening structure (1) as claimed in claim 7, wherein the hooking part (122) is strip-shaped, the linking part (121) is strip-shaped, the other end of the linking part (121) is connected to a middle of the hooking part (122), and the hooking part (122) is divided into two equal regions by the linking part (121).

9. The nonwoven fabric fastening structure (1) as claimed in claim 7, wherein the hooking part (122) is cross-shaped and has two faces, the linking part (121) is column-shaped, and the other end of the linking part (121) is connected to a center of one of the faces of the hooking part (122).

10. The nonwoven fabric fastening structure (1) as claimed in claim 7, wherein the hooking part (122) is strip-shaped and has two ends, the linking part (121) is strip-shaped, and the other end of the linking part (121) is connected to one of the ends of the hooking part (122).
